# EUROPEAN PATENT APPLICATION

(11) **EP 4 197 523 A1**
(43) Date of publication of application: **21.06.2023**
(21) Application number: 21215746.5
(22) Date of filing: 17.12.2021
(51) Int. Cl.: A61K 8/73, A61K 8/9717, A61K 8/99, A61P 17/08, A61P 17/10, A61Q 5/00, A61Q 19/00, A61K 9/00, A61K 47/10, A61K 47/14, A61K 47/26, A61K 35/74, A61K 9/19

(54) **A FREEZE-DRIED COMPOSITION AND PREPARATION THEREOF**

(71) Applicant: DSM IP Assets B.V., 6411 TE Heerlen (NL); S-Biomedic NV, 2340 Beerse (BE)
(72) Inventor: The designation of the inventor has not yet been filed
(74) Representative: Berg, Katja

(57) **Abstract**

The present invention generally relates to the field of skin care. Particularly, the invention relates to a topical composition comprising a freeze-dried composition of at least one *C*. *acnes* strain and its use for treating or preventing acne in a human subject. The invention also provides a process for preparing such freeze-dried compositions.

## Description

### Technical field

The present invention generally relates to the field of skin care. Particularly, the invention relates to a topical composition comprising a freeze-dried composition of at least one C. *acnes* strain and its use for treating or preventing acne in a human subject. The invention also provides a process for preparing such freeze-dried compositions.

### Background of the Invention

Acne (common term for Acne vulgaris) is a common inflammatory chronic skin problem, affecting the pilosebaceous units of hair follicles. About 85% of adolescents and young adults are estimated to have been affected by this disease. Acne generally manifests in inflamed papules, pustules or nodules. The inflammation may furthermore be associated with reddening, swelling and pressure pain.

It has been found that acne is highly correlated with the presence of certain (in the following called pathogenic) *Cutibacterium acnes* (C. *acnes)* strains, while others, in the following referred to as non-pathogenic C. *acnes* strains, do not lead to any adverse skin effects Thus, it was recently proposed to replace pathogenic C. *acnes* strains with non-pathogenic C. *acnes* strains on acne skin to reduce the degree and/ or severity of acne (see: A. Karoglan et al., Acta Derm Venereol 2019, 99:1253-1257). In this context, WO-2016172196 proposes an approach to treat acne which involves the consecutive steps of topically administering a disinfectant or antibiotic to the skin of a subject with acne to significantly reduce the population of pathogenic C. *acnes,* followed by topically administering one or more live, non-pathogenic C. *acnes* strain(s) to said skin. This treatment results in the non-pathogenic C. *acnes* strains becoming part of the natural skin microbiome and can thus be used to treat or prevent acne as well as to maintain skin in a condition where it is free of acne.

WO-2018073651 discloses a composition for topical administration to the skin comprising two or more different live non-pathogenic C. *acnes* strains, wherein the biomass is collected by centrifugation, resuspended in soy peptone and which suspension is then formulated as a gel.

The product form as outlined in WO-2018083651 is however not readily suitable for incorporation into a great variety of common cosmetic product forms such creams and lotions. Thus, there is an ongoing demand in the industry for an alternate product, which is easy to handle, to be stored and to be shipped while exhibiting a high viability, i.e. a viability of at least 1×10⁹ CFU/g and processes for the preparation of such product forms.

Surprisingly, it has now been found that the viability of a freeze-dried composition suitable for us in topical compositions to treat acne can be significantly improved when the biomass is isolated by microfiltration. It has furthermore been found that the viability can be further improved by a subsequent washing step. In addition, the washing step furthermore surprisingly reduces the moisture content after freeze drying and storage which is beneficial.

### Summary of the Invention

The present invention provides topical compositions comprising a freeze-dried composition comprising at least one non-pathogenic C. *acnes* strain with high viability and stability of the strain.

The present invention also provides a process for producing such freeze-dried composition.

The present invention also provides a freeze-dried composition comprising at least one non-pathogenic C. *acnes* strain which obtainable by the process above.

The present invention further provides use of said freeze-dried composition, respectively topical compositions comprising said freeze-dried compositing for treating or preventing acne in a human subject.

### Detailed Description of the Invention

The present invention provides a process for producing a freeze-dried composition comprising a non-pathogenic C. *acnes* strain for use in topical compositions to treat and/ or prevent acne in a human subject.

Particularly, the present invention provides a process for producing a freeze-dried composition comprising at least one non-pathogenic C. *acnes* strain, said process comprising the following steps:
a) concentrating the bacterial cells of the at least one non-pathogenic C. *acnes* strain from a fermentation broth to obtain a biomass concentrate comprising said cells;
b) washing said biomass concentrate to obtain a washed biomass concentrate;
c) formulating the washed biomass concentrate with an aqueous solution comprising at least one cryoprotectant to obtain a washed biomass formulation; and
d) freeze drying said washed biomass formulation to obtain the freeze-dried composition comprising the at least one non-pathogenic C. *acnes* strain.

Surprisingly, the inventors of the present invention discovered that the washing step significantly improves the recovery of viable cells as well as reduces the moisture content in the obtained freeze-dried compositions.

In the present invention, the term "non-pathogenic C. *acnes* strain" refers to strains which exhibit a slow or negligible conversion or degradation of cis-9, cis-12 linoleic acid in media, for example, C. *acnes* strain type I such as IA (i.e., IA₁ and IA₂) and IB, and type II. Examples of non-pathogenic C. *acnes* strains according to the present invention include but are not limited to the C. *acnes* strains D1, A5, C3, H1, H2, H3, K1, K2, K4, K6, K8, K9, L1 and F4 as outlined and disclosed in WO-2016172196. Preferably, the non-pathogenic C. *acnes* strain of the present invention is selected from the group consisting of the C. *acnes* strains D1, A5, C3, H1, H2, H3, K1, K2, K4, K6, K8, K9, L1 and F4. More preferably, the non-pathogenic C. *acnes* strain(s) of the present invention is (are) selected from the C. *acnes* strains C3 and/ or K8.

In the present invention, the term "viability" refers to the concentration of viable cells in a sample such as in the freeze-dried composition according to the present invention and can be expressed by "CFU/g" or "CFU/ml" of the sample.

In the present invention, the term "fermentation broth" refers to the mixture obtained after cultivating the non-pathogenic C. *acnes* strain(s) as e.g. outlined in WO-2018073651A. It is well understood by a person skilled in the art that the fermentation broth, next to the C. *acnes* strain(s), further comprises ingredients commonly present in fermentation broths such as water, nutrients such as amino acids, organic acids such as propionic acid, carbohydrates, various vitamins, growth factors, which are required by the strain to grow as well as products formed by the microorganisms itself during cultivation.

The term "skin" as used in this document, is meant to include the external surface of mammals, especially humans and includes the skin and the scalp. Preferred skin in all embodiments of the present invention is facial and body skin such as most preferably facial skin.

The term "prevention" as used herein refers to lessening the risk of developing acne.

The term "treatment" as used herein refers to an amelioration of symptoms, delaying the onset and/ or reduction of the duration of acne. The treatment can be prophylactic (cosmetic) or therapeutic. Preferably, the treatment is prophylactic.

In all embodiments of the present invention, preferably each strain is cultivated separately and accordingly the freeze-dried composition obtained according to the process of the present invention as outlined above preferably contains only one C. *acnes* strain such as in particular the C. *acnes* strain C3 or the C. *acnes* strain K8. It is, however, nevertheless not excluded according to the present invention that the fermentation broth used in the freeze drying step d) contains two or more C. *acnes* strains, e.g. by co-cultivation thereof or by combining two or more fermentation broths, each comprising one (or more) non-pathogenic C. *acnes* strain(s) thus obtaining a freeze-dried composition comprising two or more non-pathogenic C. *acnes* strains. It is furthermore understood that freeze-dried compositions according to the present invention encompassing two or more non-pathogenic C. *acnes* strains can also be obtained by admixing two or more freeze-dried compositions obtained in step d), each of which comprising one non-pathogenic C. *acnes* strain, which is preferred in all embodiments of the present invention.

In all embodiments of the present invention, the fermentation broth used in step a) according to the present invention preferably contains about 10⁸-10¹² CFU/g, preferably 10⁹-10¹¹ CFU/g of the non-pathogenic C. *acnes* strain(s).

In all embodiments of the present invention, it is further advantageous if the fermentation broth has a biomass volume (i.e. the pellet volume obtained after centrifugation of a sample thereof) selected in the range from 1 to 5 volume-%, preferably in the range of 1.5 to 3 volume-%, based on the total volume of the fermentation broth.

After fermentation and before concentration, the fermentation broth is generally collected according to standard methods in the art, e.g. by transferring it into a vessel. Particularly, the fermentation broth in the present invention may be collected at about 0-20 °C, Preferably, at 5-15 °C, more preferably at 6-12 °C such as at 8 °C and then transferred, e.g., into a vessel tank, for concentration.

The concentration in step a) can be done by standard methods in the art such as by centrifugation or filtration.

The inventors of the present invention furthermore discovered that a concentration by microfiltration in step a) preserves almost all the viable non-pathogenic C. *acnes* strain in the fermentation broth and significantly increases the viability thereof in the freeze-dried composition upon storage thereof.

In all embodiments of the present invention, the concentration in step a) is preferably done by microfiltration, more preferably by cross-flow microfiltration, most preferably using (preferably ceramic) membrane filters. Even more preferably, said membrane filters have a retention (pores size) selected in the range from 0.1 to 10 µm, preferably in the range from 0.25 to 0.75 µm, most preferably in the range from 0.4 to 0.5 µm. Such membranes are well known in the art and are e.g. commercially available at TAMI.

It is well known to a person skilled in the art, that the biomass concentrate obtained by said microfiltration is generally characterized by being a suspension of the biomass in aqueous media. According to the present invention, the microfiltration may be performed at a temperature of 0-20 °C, preferably 5-15 °C such as 8, 9, 10, 12 and 15 °C, under the pressure of 0.1-10 bar, preferably 1-8 bar, more preferably 1.5-5 bar such as 1.5, 2, 2.5, 3, 3.5, 4, 4.5 and 5 bar.

In step a) of the present invention, the fermentation broth is preferably concentrated until a biomass concentrate of 5-50 wt% of the initial weight of the fermentation broth is obtained. More preferably, the fermentation broth is concentrated until a biomass concentrate of 10-35 wt%, more preferably 15-30 wt% of the weight of the initial fermentation broth is obtained. Thus, in a preferred embodiment, the biomass concentrate constitutes 5-50 wt.-% of the weight of the fermentation broth, more preferably 10-35 wt%, most preferably 15-30 wt% of the weight of the fermentation broth.

Compared to centrifugation, the microfiltration in the step a) of the present invention increases the viability of the non-pathogenic C. *acnes* strains in the freeze-dried product obtained according to the process of the present invention upon storage after 1, preferably 3 months by at least 10%, 20%, 30%, 40% 50% or more. Preferably, the microfiltration in the step a) of the present invention increases the viability of the non-pathogenic C. *acnes* strain(s) in the freeze-dried product by at least 30% compared to centrifugation. Even more preferably, the microfiltration in the step a) of the present invention increases the viability of the non-pathogenic C. *acnes* strain in the freeze-dried product by at least 40% compared to centrifugation. Most preferably, the microfiltration in step a) of the present invention increases the viability of the non-pathogenic C. *acnes* strain in the freeze-dried product by at least 50% compared to centrifugation.

The concentrated biomass obtained in the step a) is further treated by washing according to the step b) of the process of the present invention.

In the step b) of the present invention, the biomass concentrate may be washed with an aqueous solution of any organic acid which is suitable for skin care, especially for treatment or prevention of acne. Examples of the suitable organic acid include but are not limited to citric acid, retinoic acid, ferulic acid, glycolic acid, lactic acid, fruit acids, salicylic acid and hyaluronic acid. Preferably, the organic acid is citric acid. Optionally the organic acids are used in the form of a buffer solution thereof such as a citric acid buffer solution.

In all embodiments of the present invention, it is preferred that the concentrated biomass obtained in step a) is washed with 10-100 mM, preferably 20-80 mM, more preferably 30-70 mM such as 40, 50 or even 60 mM aqueous solution of citric acid or a citric acid buffer solution (comprising citric acid and sodium citrate).

In all embodiments of the present invention it is furthermore preferred that in step b) of the present invention, the aqueous (buffer) solution of the organic acid is used in an amount of 1-10 times, preferably 2-8 times, more preferably 3-6 times such as 3, 4, 5 or 6 times of the weight of the concentrated biomass.

In step b) of the present invention, the concentrated biomass may be washed in batches or continuously. In some embodiment, the concentrated biomass is washed with aqueous citric acid in batches. In other embodiments, the concentrated biomass is washed with aqueous citric acid continuously. In case of a continuous washing, preferably the aqueous citric acid is added to the biomass concentrate and subjected again to cross-flow microfiltration.

In all embodiments of the present invention the washing is preferably continued until the biomass concentrate comprises less than 10 wt.-% of acetic acid, preferably less than 5 wt.-%, most preferably less than 3 wt.-%, such as less than 2, 1 or 0.5 wt.-%, based on the total weight of the biomass concentrate.

In case of continuous washing it is furthermore preferred in all embodiments of the present invention, that the aqueous solution of the organic acid is added to the biomass concentrate obtained in step a) (to obtain a diluted biomass concentrate) and subjected again to (cross-flow) microfiltration, more preferably until the content of the non-pathogenic C. acnes strain in the obtained washed biomass concentrate is (again) at least 1×10¹¹ CFU/g, preferably at least 1×10¹¹ CFU/g, while most preferably almost all (more than 90%, preferably more than 95%, most preferably more than 96%) of the viable cells are recovered.

Alternatively, in case of continuous washing, the aqueous solution of the organic acid is added to the biomass concentrate obtained in step a) (to obtain a diluted biomass concentrate) and then (again) subjected to (cross-flow) microfiltration until the weight of the washed biomass concentrate is less than 25 wt.-%, preferably less than 20 wt.-% of the weight of the initial fermentation broth. Even more preferably the weight of the washed biomass concentrate is 10 to 20 wt.-%, even more preferably 10 to 15 wt.-% of the initial weight of the fermentation broth.

In step b) of the present invention, the biomass concentrate may be washed at a temperature of 0-20 °C, preferably of 5-15 °C such as of 8, 9, 10, 12 and 15 °C, under a pressure of 0-10 bar, preferably of 1-8 bar, more preferably of 1.5 to 5 bar such as of 1.5, 2, 2.5, 3, 3.5, 4, 4.5 and 5 bar.

In step c) of the present application, the concentrated biomass obtained in step b) is formulated by admixing cryoprotectants into the biomass concentrate to obtain a washed biomass formulation.

In the present invention, the cryoprotectants may be selected from the group consisting of alcohols such as glycerol, propylene glycol and ethylene glycol; sugars such as glucose, galactose, lactose, sucrose, maltose, fructose, trehalose, raffinose, mannitol and sorbitol; polymers such as polyethylene glycol (PEG), polyvivyl pyrrodidone (PVP), skim milk, gelatin, protein and protein hydrolysate, peptides, yeast, broth, dextrin, maltodextrin, methylcellulose, povidone, serum and peptone; salts such as sodium sulfate, calcium lactate, sodium glutamate, sodium chloride, potassium chloride, sodium thiosulfate, trisodium citrate, ammonium acetate and ammonium chloride; acids such as citric acid, phosphoric acid, glutamic acid, tartaric acid, amino acid and ethylenediaminetetraacetic acid (EDTA); bases such as ammonium hydroxide, sodium hydroxide and sodium bicarbonate; and mixture thereof.

Preferably, in all embodiments, the cryoprotectants are selected from the group consisting of glucose, galactose, lactose, sucrose, maltose, fructose, trehalose, raffinose, mannitol, sorbitol, polyethylene glycol (PEG), polyvivyl pyrrodidone (PVP), skim milk, gelatin, protein and protein hydrolysate, peptides, yeast, broth, dextrin, maltodextrin, methylcellulose, povidone, serum, peptone, sodium sulfate, calcium lactate, sodium glutamate, sodium chloride, potassium chloride, sodium thiosulfate, trisodium citrate, ammonium acetate, ammonium chloride, citric acid, phosphoric acid, glutamic acid, tartaric acid, amino acid, ethylenediaminetetraacetic acid (EDTA) and ammonium hydroxide; and mixtures thereof.

More preferably, in all embodiments of the present invention the cryoprotectants are selected from the group consisting of glucose, sucrose, trehalose, raffinose, mannitol, sorbitol, polyethylene glycol (PEG), dextrin, maltodextrin, peptone, sodium glutamate, glutamic acid, sodium chloride, trisodium citrate, and ammonium hydroxide; and mixtures thereof.

Most preferably, in all embodiments of the present invention, the cryoprotectants are selected from the group consisting of sucrose, trehalose, raffinose, mannitol, polyethylene glycol (PEG), maltodextrin, peptone, sodium glutamate, sodium chloride, trisodium citrate, glutamic acid and sodium bicarbonate; and mixtures thereof.

In one advantageous embodiment, the cryoprotectants are a combination of two or more of sucrose, maltodextrin, glutamic acid, sodium glutamate and trisodium citrate.

In another particular advantageous embodiment, the cryoprotectants are a combination of sucrose, maltodextrin, sodium glutamate and trisodium citrate.

In a further particular advantageous embodiment, the cryoprotectants are a combination of sucrose, maltodextrin and glutamic acid.

As anticipated by any person skilled in the art, an aqueous solution of the cryoprotectants is prepared before mixing with the washed biomass concentrate according to the present invention. Preferably, said aqueous solution should be clear and exhibit no precipitation during preparation and storage. Preparation of such aqueous solutions and mixing can be done according to the procedures known to the skilled person in the art.

In all embodiments of the present invention, the formulation obtained in the step c) may contain 1-20 wt%, preferably 5-15 wt%, more preferably 6-12 wt% such as 6, 7, 8, 9, 10, 11 and 12 wt% of cryoprotectant(s), based on the total weight of the formulation.

After the formulation is prepared, it is freeze-dried in the step d) according to the present invention.

Freeze drying (lyophilization) is a very well-established method for the archiving and long-term storage of strains. The approaches are used vary widely, but they all follow the standard process associated with lyophilization, namely freezing the sample, applying a high vacuum, warming the sample under vacuum to cause water sublimation, driving off excess water through a drying phase, and finally sealing the sample to prevent water uptake. A basic freeze-drying process can be divided into three stages: freezing, primary drying, and secondary drying.

### Freezing

In the freezing stage, the formulation of the present invention may be frozen to -10 °C to -50 °C, preferably -20 °C to -40 °C such as -25 °C, -30 °C and -40 °C.

In the present invention, the formulation may be frozen rapidly, for example, by putting the formulation in a tray which is precooled at -20 °C, at -25 °C or at -40 °C.

In the present invention, the formulation is preferably frozen gradually, for example, by cooling down at 1 °C/min, to -20 °C, -25 °C or -40 °C.

In one embodiment of the present invention, the formulation is frozen rapidly to -20 °C, then cooled down at 1 °C/min to -40 °C.

### Primary drying

Once the formulation is frozen, a vacuum is applied to remove the frozen water. In this stage, the formulation of the present invention may be subjected to a vacuum under 1 mbar, preferably under 0.8 mbar, more preferably under 0.6 mbar, and the most preferably under 0.5 mbar such as 0.1, 0.2, 0.3, 0.4 and 0.5 mbar.

The temperature for such primary drying may be at from -10 °C to -50 °C, preferably from -20 °C to -40 °C such as -25 °C, -30 °C and -40 °C.

As understood by any person skilled in the art, the length of time required for the primary drying could be determined by comparative pressure measurement which are known in the art. In the present invention, the primary drying may take 30-80 hours, preferably 40-60 hours.

### Secondary drying

After the above the primary drying, the formulation is further subjected to a second drying to force out the residual water. In this stage, the vacuum is maintained while the temperature is increased.

As understood by any person skilled in the art, it is important not to over dry the formulation as this can be detrimental, and the use of higher temperatures is also not recommended for the same reason. In the present invention, the temperature of the formulation is increased to above 10 °C, such as 20 °C and 30 °C, but less than 50 °C. Preferably, the formulation is warmed to ambient temperature.

As also understood by any person skilled in the art, it is good to increase the temperature of the formulation of the present invention gradually. Preferably, the temperature of the formulation of the invention is increased to 20 °C in 2-20 hours, preferably in 5-15 hours, more preferably in 6-10 hours such as 6, 6.5, 7, 7.5, 8, 8.5, 9, 9.5 and 10 hours.

Preferably, the formulation of the present invention is maintained for additional 1-10 hours after it is warmed to the desired temperature. More preferably, the formulation of the present invention is maintained for additional 2-15 hours, even more preferably 3-10 hours such as 3, 4, 5, 6, 7, 8, 9 and 10 hours at 10 °C, 20 °C or 30 °C.

Standard freeze-drying devices can be used for the above freeze-drying operations, such as the Lyovac^{™} devices from GEA (Berlin, Germany), the Gamma 2-20 Freeze dryer LCM-1 from Christ (Osterode am Harz, Germany), the Christ Martin^{™} Alpha 1-2 Lyophilisator from Fisher Scientific GmbH (Schwerte, Germany), and the Virtis Genesis 35L Pilot Freeze dryer from SP Scientific (PA, US). Any person skilled in the art could operate any of the devices to perform the freeze-drying of the present invention according to the instructions of the manufacture.

After freeze drying of the formulation, a cake of a freeze-dried composition is obtained. It can be crushed and stored in a sealed container such as an aluminum bag or a bottle for further use.

In a preferable embodiment, the present invention provides a process for producing a freeze-dried composition comprising at least one non-pathogenic C. *acnes* strain, comprising the following steps:
a) concentrating the bacterial cells of the at least one non-pathogenic C. *acnes* strain from a fermentation broth to obtain a biomass concentrate;
b) washing the biomass concentrate to obtain a washed biomass concentrate;
c) formulating the washed biomass concentrate with an aqueous solution comprising at least one cryoprotectant to obtain a biomass formulation; and
d) freeze drying the biomass formulation to obtain the freeze-dried composition,
wherein the concentration in step a) and the washing in step b) is performed by cross-microfiltration.

According to the process of the present invention, the obtained freeze-dried composition has a high viability of the non-pathogenic C. *acnes* strain. Particularly, the obtained freeze-dried composition has a viability of at least 1×10⁹ CFU/g of the non-pathogenic C. *acnes* strain, preferably of at least 1×10¹⁰ CFU/g, more preferably of at least 5×10¹⁰ CFU/g, of at least 6×10¹⁰ CFU/g, of at least 7×10¹⁰ CFU/g, of at least 8×10¹⁰ CFU/g, of at least 9×10¹⁰ CFU/g or of at least 1×10¹¹ CFU/g.

In addition, the process of the present invention provides a high stability (storage viability) of the strain in the obtained freeze-dried composition. Particularly, the freeze-dried composition produced according to the process of the present invention maintains at least 80%, preferably 85%, more preferably 90% viability of the strain after 4 weeks of storage at room temperature, or at least 60%, preferably 65%, more preferably 70% viability of the strain after 4 weeks of storage at 37 °C.

Step b) of the process according to the present invention provides additional advantages, for example, the viability of the non-pathogenic C. *acnes* strain is preserved better compared to the process without the step b). In addition, the step b) provides low residual moisture content (less than 3 wt%, preferably less than 2wt%, more preferably less than 1.5wt%, even more preferably less than 1.2wt%) in the freeze-dried composition of the present invention (as measured with a Sartorius moisture analyser (Sartorius AG, Germany)

Accordingly, as a second aspect of the present invention, the present invention is concerned with a freeze-dried composition obtainable (obtained) by the above process according to the invention and as outlined herein and in the claims.

Particularly, the present invention provides a freeze-dried composition comprising at least one non-pathogenic C. *acnes* strain, wherein the viability of the at least one non-pathogenic C. *acnes* strain, preferably of one or more of C. *acnes* strain type I such as IA (i.e., IA₁ and IA₂) and IB, and type II, most preferably of C. *acnes* strain C3 and/ or the C. *acnes* strain K8 is at least 1×10⁹ CFU/g of the composition.

Advantageously, in all embodiments of the present invention, in the freeze-dried composition of the present invention, the viability of the non-pathogenic C. *acnes* strain(s), preferably of one or more of C. *acnes* strain type I such as IA (i.e., IA₁ and IA₂) and IB, and type II, most preferably of C. *acnes* strain C3 and/ or the C. *acnes* strain K8 is at least 1×10¹⁰ CFU/g, at least 5×10¹⁰ CFU/g, at least 6×10¹⁰ CFU/g, at least 7×10¹⁰ CFU/g, at least 8×10¹⁰ CFU/g, at least 9×10¹⁰ CFU/g and at least 1×10¹¹ CFU/g, of the composition. More preferably, the viability of the non-pathogenic C. *acnes* strain in the freeze-dried composition of the present invention is from 1×10¹⁰ CFU/g to 1×10¹³ CFU/g, more preferably from 5×10¹⁰ CFU/g to 8×10¹² CFU/g, the most preferably from 1×10¹¹ CFU/g to 5×10¹² CFU/g such as 3×10¹¹ CFU/g, 5×10¹¹ CFU/g, 6×10¹¹ CFU/g, 7×10¹¹ CFU/g, 8×10¹¹ CFU/g, 9×10¹¹ CFU/g and 1×10¹² CFU/g of the composition. Preferably, the composition of the present invention contains at least 7×10¹¹ CFU/g of the C. *acnes* strain C3 and/or at least 6×10¹¹ CFU/g of the C. *acnes* strain K8.

Advantageously, in the freeze-dried composition of the present invention, the residual moisture is less than 3 wt%, preferably less than 2wt%, more preferably less than 1.5wt%, most preferably less than 1.2wt%.

Preferably, the freeze-dried composition of the present invention maintains at least 80%, preferably 85%, more preferably 90% viability of the strain(s) after 4 weeks of storage at room temperature, or at least 60%, preferably 65%, more preferably 70% viability of the strain(s) after 4 weeks of storage at 37 °C.

The freeze-dried composition of the present invention can be used in for treating or preventing (prophylaxis of) acne in a human subject.

Accordingly, as a third aspect, the present invention provides use of the freeze-dried composition of the present invention or a freeze-dried composition prepared according to the process according to the present invention for treating or preventing acne in a human subject and optionally appreciating the effect.

The present invention also provides a method for treating or preventing acne in a human subject, comprising topically administering to the subject a topical composition comprising the freeze-dried composition of the present invention.

Accordingly, as an additional aspect, the present invention also relates to a topical composition comprising the freeze-dried composition with all the preferences and definitions as outlined herein.

To be used in the topical composition, the viability of the freeze-dried composition according to the present invention is preferably adjusted to about 1×10¹⁰ CFU/g (if necessary) by admixture with maltodextrin (calibrated freeze-dried composition). Furthermore, one or more of the freeze-dried compositions comprising different C. acnes strains can be admixed, such as preferably, a freeze-dried composition comprising C. *acnes* C3 and a freeze-dried composition comprising C. *acnes* K8. Said admixing can take place either before or after the CFU/g adjustment.

As the topical compositions according to the invention are intended for topical application, it is well understood that they comprise a physiologically acceptable medium, i.e. a medium compatible with keratinous substances, such as the skin, mucous membranes, and keratinous fibers. In particular, the physiologically acceptable medium is a cosmetically respectively dermatologically acceptable carrier

The term 'cosmetically/ dermatologically acceptable carrier' (also referred to herein as carrier) refers to all vehicles/ carriers conventionally used in cosmetic or dermatological compositions, i.e. which are suitable for topical application to the keratinous tissue, have good aesthetic properties, are compatible with the actives present in the composition, and will not cause any unreasonable safety or toxicity concerns. Such carriers are well-known to one of ordinary skill in the art.

The exact amount of carrier will depend upon the actual level of the freeze-dried composition and any other optional ingredients that one of ordinary skill in the art would classify as distinct from the carrier (e.g., other active ingredients).

In an advantageous embodiment, the topical compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier does not comprise any water, i.e. the topical compositions according to the present invention are anhydrous topical compositions.

In another particular advantageous embodiment, the topical composition according to the present invention consists of the freeze-dried composition according to the present invention with all the definitions and preferences as given herein, at least one oil, at least one oil soluble antioxidant such as e.g. tocopherol. The at least one oil is preferably selected from fatty alcohols and/ or fatty acid glycerides.

Particularly suitable topical compositions according to the invention are leave-on products, and include any product applied to the human body. The composition can be in the form of a liquid, lotion, cream, foam, or applied with an implement or via a face mask, pad or patch. Non-limiting examples of such compositions include leave-on skin lotions and creams, foundations, mascara, sunless tanners and sunscreen lotions.

The topical compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The amount of the (preferably calibrated) freeze-dried composition in the topical composition according to the present invention can be adjusted by a person skilled in the art and is preferably selected in the range of 0.1 to 10 wt.-%, preferably 0.5 to 7.5 wt.-%, most preferably 0.75 to 5 wt.-%, such as from 1 to 4 wt.-%, based on the total weight of the composition.

The following examples are provided to further illustrate the compositions and effects of the present invention. These examples are illustrative only and are not intended to limit the scope of the invention in any way.

### Examples

### CFU analysis

The CFU analysis was performed as follows: 100 µL sample was taken and 10⁻¹ to 10⁻⁹ dilutions were prepared with physiological water in series. 100 µL of each 10⁻⁷ to 10⁻⁹ dilutions was plated on TSA plates which were incubated in anaerobic jars at 37 °C using the Anaerocult incubation system for at least 4 days. Counting (plates between 30-300 colonies) was performed manually.

The CFU analysis of the freeze-dried composition obtained by the process of the present invention was performed as follows: 0.3 g powderwas resuspended in 9.7 mL sterile physiological water (gravimetric dilution). Serial dilution, plating and incubation was performed as described above.

### Collection, microfiltration and washing

Wash solution: 50 mM citric acid buffer solution in water (pH) was prepared the day before and stored in 20 L jerry cans at 4 °C.

The respective fermentation broths, kept at 8 °C at the end of fermentation, were harvested and hygienically transferred into an internal vessel tank. Afterwards the fermentation broth were subjected to cross-microfiltration. The equipment was employed for the concentration and then washing with the citric acid buffer solution. For comparative results, the C. *acnes* C3 batch was split and also worked up by centrifugation and washing by resuspension/ centrifugation of the cell pellet in the citric acid buffer twice.

### Biomass formulation

The aqueous cryoprotectant solution was prepared on weight basis as shown in Table 1 and made up to the final volume with water, after pH adjustment. Specifically, after having added the components one by one and waiting in between until the previous component was fully dissolved, the pH was adjusted to 6 using a solution of 50 mM citric acid. The prepared solution was sterilized and stored at 4°C prior to usage.

**Table 1: Cryoprotectant solution**

| **Component** | **Concentration (g/L)** |
|---|---|
| Tri sodium citrate (dihydrate.) | 10 |
| Sucrose | 75 |
| Sodium Glutamate (monohydrate.) | 90 |
| Maltodextrin | 225 |

The formulated biomass concentrates were prepared by adding to the (washed) biomass concentrate ½ weight of the cryoprotectant solution (weight ratio biomass/ cryoprotectant solution 2:1).

### Freeze-drying

Freeze drying of (washed) biomass formulations was performed in Virtis Genesis 35L Pilot Freeze dryer from SP Scientific (PA, US) as follows:
- Start: shelf at 3°C.
- Freezing phase: lower to -40 °C at 1°C/min.
- Primary drying phase: pull vacuum to 0.1 mbar, increase to -25°C in 15 min.
- Primary drying phase: hold -25 °C for 58 hours
- Secondary drying: ramp to 20 °C in 7,5 hours, and hold at 20 °C for 4 hours.

After freeze-drying, each tray was placed into a zipper plastic bag. The cake was removed from the tray by manipulating the bag and by applying some pressure to the bottom of the tray. The plastic bag was opened to remove the tray and the cake was manually crushed.

### Results

Table 2 and Table 3 show an overview of the process parameters during the microfiltration operation of the C. *acnes* strain K8 batch and the C. *acnes* strain C3 batch, respectively.

**Table 2: Overview of microfiltration/ washing of the C. acnes strain K8 batch**

| **Process steps** | **Parameter** | **Value** |
|---|---|---|
| Concentration | Initial Volume (Kg) | 97.3 |
| | Pressure (bar) | 3 |
| | Filtrate flow rate (Kg/h) | 60 |
| | End volume - biomass concentrate (Kg) | 22.4 |
| Washing | Citric acid buffer (pH6) added (Kg) | 60 |
| | Pressure (bar) | 3 |
| | Filtrate flow rate (Kg/h) | 60 |
| | End volume - washed biomass concentrate (Kg) | 14.14 |

**Table 3: Overview of microfiltration/ washing of the C. acnes strain C3 batch**

| **Process steps** | **Parameter** | **Value** |
|---|---|---|
| Concentration | Initial Volume (Kg) | 99.2 |
| | Pressure (bar) | 3 |
| | Filtrate flow rate (Kg/h) | 37 |
| | End volume - biomass concentrate | 25 |
| Washing (optional) | Citric acid buffer (pH6) added (Kg) | 66 |
| | Pressure (bar) | 3 |
| | Filtrate flow rate (Kg/h) | 35 |
| | End volume - washed biomass concentrate (Kg) | 12.78 |

CFU analysis was performed on the biomass concentrates, the washed biomass concentrates as well as on the freeze-dried compositions derived from washed and non-washed biomass concentrates. The results illustrate that there is no recovery loss during microfiltration for both C. *acnes* strain K8 (Table 4) and C. *acnes* strain C3 (Table 5) as well as a significant improvement in the recovery for freeze-dried compositions using a washed biomass concentrate compared to the use of a non-washed biomass concentrate (Table 5).

**Table 4: CFU analysis of C. acnes strain K8 batch**

| | **Kg** | **CFU/g** | **Total CFU** | **Recovery** |
|---|---|---|---|---|
| Fermentation broth | 97.3 | 1.87×10¹⁰ | 1.82×10¹⁵ | 100% |
| Washed biomass concentrate | 14.14 | 1.25×10¹¹ | 1.77×10¹⁵ | 97% |
| Freeze-dried composition | 3.09 | 6.43×10¹¹ | 1.99×10¹⁵ | 109% |

**Table 5: CFU analysis of C. acnes strain C3 batch**

| | **Kg** | **CFU/g** | **Total CFU** | **Recovery** |
|---|---|---|---|---|
| Fermentation broth | 99.22 | 3.37×10¹⁰ | 3.34×10¹⁵ | 100% |
| Washed biomass concentrate | 12.78 | 2.70×10¹¹ | 3.92×10¹⁵ | 117% |
| Freeze-dried composition (non-washed) | 4.9 | 2.1×10¹¹ | 1.32×10¹⁵ | 39% |
| Freeze-dried composition (washed) | 4.5 | 7.08×10¹¹ | 3.58×10¹⁵ | 107% |

### Residual moisture

To investigate storage stability, residual moisture content analysis was performed 1 month after freeze-drying of the C. *acnes* strain C3 batch using the Sartorius moisture analyser (1g/ 100°C) (Sartorius AG, Germany). The results illustrate that the washing step also lowered the residual moisture of the freeze-dried composition (see table 6), reflecting improved stability.

**Table 6: residual moisture of the freeze-dried powder of C. acnes strain C3**

| | Residual Moisture Level (%) |
|---|---|
| Freeze-dried composition - non washed | 3.77 |
| Freeze-dried composition - washed | 2.05 |

### Comparative experiment microfiltration vs. centrifugation

After end of fermentation and cooling down the respective broths of C. *acnes* C3 and C. *acnes* K8 are harvested, concentrated, washed, formulated and freeze-dried as outlined above. Subsequently the freeze-dried compositions of the C. *acnes* strains C3 and K8 were blended and the viability of the blend was adjusted with maltodextrin to be in the range of about 1×10¹⁰ (in the following referred to as C. *acnes* blend). The viability of said blend upon storage was investigated over time.

As can be retrieved from the data outlined in table 7 below, the microfiltration led to a significant improvement in the viability upon storage (accelerated stability test at 40°C).

**Table 7: cell viability upon storage**

| **Time [months]** | | **Microfiltration** | | **Centrifugation** | |
|---|---|---|---|---|---|
| 0 | 1.08×10¹⁰ | | 100% | 1.01×10¹⁰ | 100% |
| 1 | 1.15×10¹⁰ | | 107% | 4.23×10⁹ | 42% |
| 3 | 1.15×10¹⁰ | | 107% | 7.14×10⁸ | 7% |

### Cosmetic composition

In order to illustrate formulation compatibility, the C. *acnes* blend was incorporated into a formulation suitable for topical application for the treatment of acne as outlined in table 8.

**Table 8: Anti-acne formulation**

| **Phase** | **raw material** | **% w/w** |
|---|---|---|
| | | |
| A | Myritol 318 (CAPRYLIC/CAPRIC TRIGLYCERIDE) | 40.00 |
| | Oilkemia 5S Polymer (CAPRYLIC/CAPRIC GLYCERIDES; POLYURETHANE-79) | 4.50 |
| | Mixed Tocopherols 95 | 0.30 |
| | Eutanol G (OCTYLDODECANOL) | Ad 100 |
| | | |
| B | C. *acnes* blend | **1.00** |

### Preparation:

Mix phase A and heat it up to 85°C under stirring. Homogenize 2 min at 14'000 rpm. Let cool down to room temperature. Weigh phase A in a sterile beaker. Add B to A and stirr for 5 min at 350 rpm with an anchor stirrer.

## Claims

1. A topical composition comprising a freeze-dried composition comprising at least one non-pathogenic C. *acnes* strain, wherein the viability of the non-pathogenic C. *acnes* strain in said composition is at least 1×10⁹ CFU/g.

2. The topical composition of claim 1, wherein the non-pathogenic C. *acnes* strain in the freeze-dried composition is selected from the group consisting of C. *acnes* strain type I such as IA (i.e., IA₁ and IA₂) and IB, and type II as well as mixtures thereof.

3. The topical composition of claim 1, wherein the non-pathogenic C. *acnes* strain in the freeze-dried composition is selected from the group consisting of C. *acnes* strains D1, A5, C3, H1, H2, H3, K1, K2, K4, K6, K8, K9, L1 and F4 as well as mixtures thereof.

4. The topical composition of claim 1, wherein the at least one non-pathogenic C. *acnes* strain in the freeze-dried composition is the C. *acnes* strain C3 or the C. *acnes* strain K8 as well as mixtures thereof.

5. The topical composition of any one or more of claims 1-4, wherein the viability of the at least one non-pathogenic C. *acnes* strain in the freeze-dried composition is at least 1×10¹⁰ CFU/g.

6. The topical composition of one or more of claims 1-5, wherein the moisture content of the freeze-dried composition (before incorporation in the topical composition) is less than 3 wt%, preferably less than 2 wt%, more preferably less than 1.5 wt%, most preferably less than1.2 wt.-%, based on the total weight of the freeze-dried composition.

7. The topical composition of one or more of claims 1-6 for use in the treatment or prevention of acne

8. A process for producing a freeze-dried composition according to anyone of the preceding claims, comprising the steps of:
a) concentrating the bacterial cells of the at least one non-pathogenic C. *acnes* strain from a fermentation broth to obtain a biomass concentrate comprising said cells;
b) washing said biomass concentrate to obtain a washed biomass concentrate;
c) formulating the washed biomass concentrate with an aqueous solution comprising at least one cryoprotectant to obtain a washed biomass formulation; and
d) freeze drying said washed biomass formulation to obtain the freeze-dried composition comprising the at least one non-pathogenic C. *acnes* strain.

9. The process of claim 8, wherein the concentration of the fermentation broth in the step a) is performed by microfiltration, preferably by cross-microfiltration.

10. The process according to claim 8 and/ or 9, wherein the biomass concentrate is washed in step b) with an aqueous (buffer) solution of an organic acid which is selected from the group consisting of citric acid, retinoic acid, ferulic acid, glycolic acid, lactic acid, fruit acids, salicylic acid and hyaluronic acid as well as mixtures thereof, preferably citric acid.

11. The process of claim 9, wherein the biomass concentrate is washed with a 10-100 mM, preferably 20-80 mM, more preferably 30-70 mM such as 40, 50 and 60 mM aqueous (buffer) solution of citric acid.

12. The process of any one or more of claims 8-11, wherein the biomass concentrate or the washed biomass concentrate is formulated in the step c) by admixing said biomass concentrate with at least one cryoprotectant selected from the group consisting of alcohols such as glycerol, propylene glycol and ethylene glycol; sugars such as glucose, galactose, lactose, sucrose, maltose, fructose, trehalose, raffinose, mannitol and sorbitol; polymers such as polyethylene glycol (PEG), polyvivyl pyrrodidone (PVP), skim milk, gelatin, protein and protein hydrolysate, peptides, yeast, broth, dextrin, maltodextrin, methylcellulose, povidone, serum and peptone; salts such as sodium sulfate, calcium lactate, sodium glutamate, sodium chloride, potassium chloride, sodium thiosulfate, trisodium citrate, ammonium acetate and ammonium chloride; acids such as citric acid, phosphoric acid, glutamic acid, tartaric acid, amino acid and ethylenediaminetetraacetic acid (EDTA); bases such as ammonium hydroxide, sodium hydroxide and sodium bicarbonate; and mixtures thereof.

13. The process of claim 12, wherein the at least one cryoprotectant is selected from the group consisting of sucrose, maltodextrin, glutamic acid, sodium glutamate and trisodium citrate as well as mixtures thereof.

14. The process of any one or more of claims 8 to 13, wherein the washed biomass formulation in the step c) contains 1-20wt%, preferably 5-15wt%, more preferably 6-12wt% such as 6, 7, 8, 9, 10, 11 and 12wt% of the cryoprotectants, based on the total weight of the washed biomass formulation.

15. A freeze-dried composition obtainable by a process according to any one of claims 8-14.
